(19) Europäisches Patentamt
European Patent Office
Office européen des brevets

(11) Veröffentlichungsnummer : **0 115 639**
**B1**

(12) # EUROPÄISCHE PATENTSCHRIFT

(45) Veröffentlichungstag der Patentschrift : **04.03.87**

(51) Int. Cl.⁴ : **C 07 D207/16**, C 07 D209/42, C 07 D209/52

(21) Anmeldenummer : **83113193.3**

(22) Anmeldetag : **29.12.83**

(54) **Disubstituierte Prolinderivate, Verfahren zu ihrer Herstellung und ihre Verwendung.**

(30) Priorität : **07.01.83 DE 3300316**

(43) Veröffentlichungstag der Anmeldung : **15.08.84 Patentblatt 84/33**

(45) Bekanntmachung des Hinweises auf die Patenterteilung : **04.03.87 Patentblatt 87/10**

(84) Benannte Vertragsstaaten : **AT BE CH DE FR GB IT LI LU NL SE**

(56) Entgegenhaltungen :
**EP-A- 0 050 850**
**DE-A- 3 151 690**
**US-A- 4 350 704**
**CHEMICAL REVIEWS, vol. 66, 1966, Washington, A.B. MAUGER UND B. WITKOP, "Analogs and homologs of proline and hydroxyproline", Seiten 47-86**

(73) Patentinhaber : **HOECHST AKTIENGESELLSCHAFT**
**Postfach 80 03 20**
**D-6230 Frankfurt am Main 80 (DE)**

(72) Erfinder : **Henning, Rainer, Dr.**
**Rotenhofstrasse 31**
**D-6234 Hattersheim am Main (DE)**
Erfinder : **Urbach, Hansjörg, Dr.**
**Le Lavandoustrasse 41**
**D-6242 Kronberg/Taunus (DE)**

**EP 0 115 639 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Verbindungen der Formel I

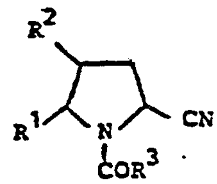

(I)

in welcher

$R^1$ und $R^2$ gleich oder verschieden sind und für geradkettiges oder verzweigtes Alkyl mit 1 bis 6 C-Atomen oder für ($C_6$ bis $C_{10}$)-Aryl, vorzugsweise Phenyl stehen oder gemeinsam für eine der Ketten $-(CH_2)_n-$ oder $-(CH_2)_p-CH=CH-(CH_2)_q$ stehen, wobei n, p und q jeweils ganzzahlig, n = 3 — 6 und (p + q) = 1 — 4 sind, und

$R^3$ geradkettiges oder verzweigtes Alkyl mit 1 bis 5 C-Atomen oder ($C_6$ bis $C_{10}$)-Aryl, vorzugsweise Phenyl bedeutet,

1-Acetyl-2-cyano-2β,3aβ,7aβ-octahydroindol, N-Acetyl-2-cyano-2β,3aβ,6aβ-octahydrocyclopenta[b]pyrrol und N-Acetyl-2-cyano-2β,3aβ,8aβ-decahydrocyclohepta[b]pyrrol ausgenommen.

Es ist bekannt, daß man disubstituierte Prolinderivate durch verschiedene klassische Methoden der Aminosäuresynthese herstellen kann. Eine Übersicht über die bekannten Arbeiten hierzu wurde von A. B. Mauger und B. Witkop publiziert (Chem. Rev. *66,* 47 (1966)). Bei den dort beschriebenen Verfahren fallen die Verbindungen zumeist als komplexe Isomerengemische an. Da bestimmte 4,5-disubstituierte Isomere, die als Ausgangsverbindungen für weitere Synthesen benötigt wurden, schlecht oder überhaupt nicht nach bekannten Verfahren und anschließende Isomerentrennung erhältich waren, stellte sich die Aufgabe, nach neuen, zu solchen Isomeren führenden Synthesewegen zu suchen.

Die erfindungsgemäßen Prolinderivate der Formel I sind wertvolle Zwischenprodukte auf einem solchen Syntheseweg. Bevorzugte Verbindungen der Formel I sind solche, in denen $R^1$ und $R^2$ gemeinsam eine der oben genannten Ketten bilden und zueinander trans-konfiguriert sind. Sind $R^1$ und $R^2$ nicht durch eine Kette miteinander verknüpft, so können diese Rest cis- oder trans-Konfiguration zueinander und zur CN-Gruppe aufweisen.

Die Verbindungen der Formel I können in den diastereomeren Formen Ia-d auftreten, deren relative Konfiguration durch die folgenden Formeln wiedergegeben werden kann.

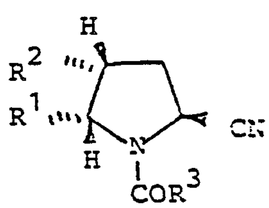

(Ia)
2β,4α,5α-Form

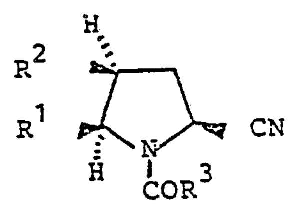

(Ib)
2β,4β,5β-Form

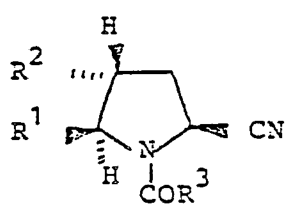

(Ic)
2β,4α,5β-Form

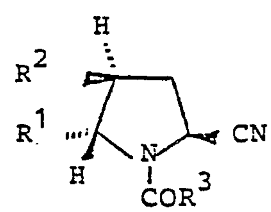

(Id)
2β,4β,5α-Form

Die Bezeichnung « α » bedeutet, daß der Substituent in der entsprechenden Position unterhalb, die Bezeichnung « β », daß er oberhalb der Ebene des Fünfrings steht. Falls $R^1$ und $R^2$ miteinander verbunden sind, ändert sich die Bezifferung entsprechend den Konventionen für bicyclische Ringsysteme.

Die Erfindung betrifft ferner ein Verfahren zur Herstellung von Verbindungen der Formel I, in der $R^1$, $R^2$ und $R^3$ die oben genannten Bedeutungen haben, das dadurch gekennzeichnet ist, daß man eine

Organoquecksilberverbindung der Formel II

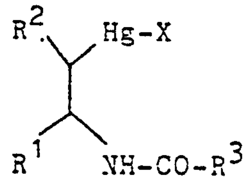

(II)

in welcher $R^1$, $R^2$ und $R^3$ die oben genannte Bedeutung haben und X Halogen, vorzugsweise Chlor oder Brom, Pseudohalogen oder Acetat bedeuten, mit 2-Chloracrylnitril und Natriumborhydrid oder Kaliumborhydrid zu einer Verbindung der Formel III

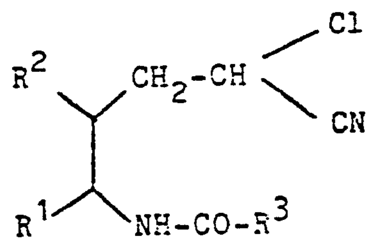

(III)

in welcher $R^1$, $R^2$ und $R^3$ die oben genannte Bedeutung haben, umsetzt.

Die Reaktion wird in einem alkoholischen Lösungsmittel, vorzugsweise Ethanol, mit einem ein- bis fünfzehnfachen, vorzugsweise einem drei- bis achtfachen Überschuß an 2-Chloracrylnitril bei —20 °C bis 60 °C, vorzugsweise bei 0 °C bis 30 °C durchgeführt.

Anschließend wird die Verbindung der Formel III durch Umsetzung mit einer geeigneten Base, vorzugsweise Natriumhydrid in einem aprotischen dipolaren Lösungsmittel wie Dimethylsulfoxid oder Dimethylformamid oder mit Kaliumcarbonat oder Kaliumhydroxid in einem aprotischen Lösungsmittel wie vorzugsweise Acetonitril gegebenenfalls unter Zusatz eines Phasentransferkatalysators, vorzugsweise Triethylbenzylammoniumchlorid bei —20 °C bis 80 °C, vorzugsweise bei 0 °C bis 40 °C zur Verbindung der Formel I cyclisiert.

Die Erfindung betrifft auch Verbindungen der Formel III, in der $R^1$, $R^2$ und $R^3$ die oben genannte Bedeutung haben.

Weiterhin betrifft die Erfindung die Verwendung von Verbindungen der Formel I, in der $R^1$, $R^2$ und $R^3$ die oben genannte Bedeutung haben, in einem Verfahren zur Herstellung von Verbindungen der Formel IV

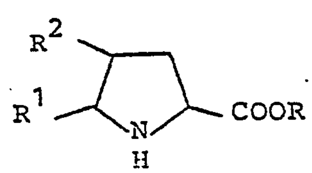

(IV)

in welcher $R^1$ und $R^2$ die oben genannten Bedeutungen haben und R für Wasserstoff, Alkyl mit 1 bis 10 C-Atomen, Aralkyl mit 7 bis 9 C-Atomen, Cycloalkyl mit 5 bis 10 C-Atomen, Alkylcycloalkyl mit 6 bis 12 C-Atomen oder Cycloalkylalkyl mit 6 bis 12 C-Atomen steht, das dadurch gekennzeichnet ist, daß die Verbindungen der Formel I einer Solvolyse mit Verbindungen der Formel ROH, in welcher R vorstehende Bedeutung hat unterworfen werden.

Bevorzugt ist die Hydrolyse. Dabei werden die Verbindungen der Formel I in Gegenwart einer starken Säure, vorzugsweise einer Mineralsäure wie Chlorwasserstoffsäure oder Bromwasserstoffsäure in wäßriger Lösung bei 20 °C bis 160 °C, vorzugsweise bei 70 °C bis 120 °C in die Aminosäuren der Formel IV, in der R Wasserstoff bedeutet, umgewandelt.

Anfallende Isomerengemische können auf den verschiedenen Verfahrensstufen (Verbindungen der Formeln III, I und IV) durch an sich bekannte Trennmethoden, wie beispielsweise durch fraktionierte Kristallisation oder Säulenchromatographie in die Isomeren aufgetrennt werden.

Die Ausgangsstoffe zur Herstellung von Verbindungen der Formel I sind aus J. Prakt. Chem. *311*, 737 (1969) sowie aus J. Org. Chem. *41*, 192 (1976) bekannt : sie entstehen durch Addition eines Quecksilbersalzes und eines Nitrils an die Doppelbindung eines Olefins der Formel V, in der $R^1$ und $R^2$ die oben genannte Bedeutung besitzen.

$$R^1—CH=CH—R^2 \qquad (V)$$

Die Addition der beiden Komponenten an die Doppelbindung erfolgt von entgegengesetzten Seiten. Daraus folgt, daß man ausgehend von cis-disubstituierten Olefinen der Formel Va

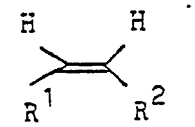

(Va)

die trans-disubstituierten Verbindungen der Formel IVa erhält,

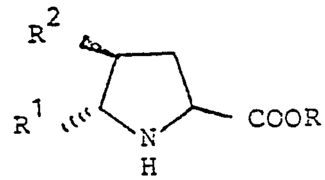

(IVa)

da die Schritte des erfindungsgemäßen Verfahrens unter Erhalt der Konfiguration an den jeweiligen Kohlenstoffatomen ablaufen. Analog erhält man aus trans-disubstituierten Olefinen der Formel Vb

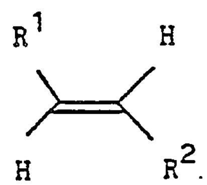

(Vb)

die cis-disubstituierten Verbindungen der Formel IVb

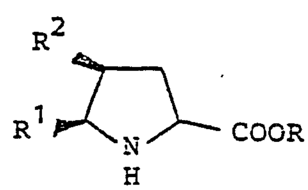

(IVb)

ausgehend von Cycloolefinen der Formel VIa bzw. VIb

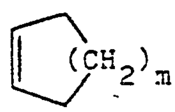

(VIa)

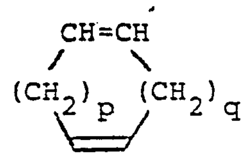

(VIb)

wobei m eine Zahl von 1 bis 4 bedeutet, erhält man bicyclische Verbindungen der Formel VIIa bzw. VIIb

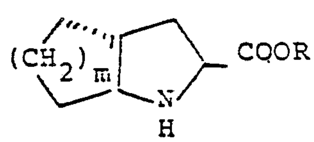

(VIIa)

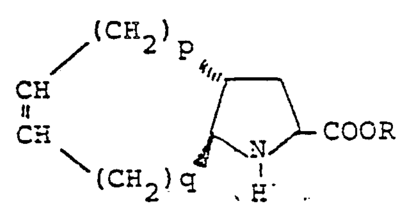

(VIIb)

deren beide Ringe transständig miteinander verknüpft sind.

Die Verbindungen der Formeln I und IV sind wertvolle Zwischenprodukte bei der Herstellung von Pharmazeutika, insbesondere von Inhibitoren des « Angiotensin Converting Enzyms » (ACE). Verbindungen dieses Typs sind beispielsweise aus der EP-A 50 800 bekannt oder sind auch Gegenstand der deutschen Patentanmeldung P 31 51 690.4.

Solche ACE-Inhibitoren sind beispielsweise substituierte Acylderivate der Formel VIII

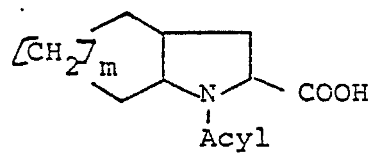

(VIII)

in welcher m wie vorstehend definiert ist und Acyl z. B. für einen Rest der Formel IX

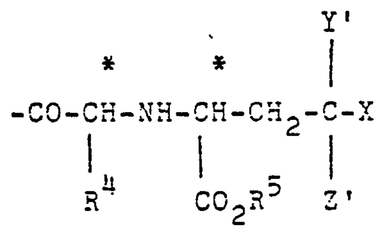

(IX)

worin

$R^4$ = Wasserstoff, ($C_1$-$C_6$)-Alkyl, das gegebenenfalls durch Amino, ($C_1$-$C_4$)-Acylamino oder Benzoylamino substituiert sein kann, ($C_2$-$C_6$)-Alkenyl, ($C_5$-$C_9$)-Cycloalkyl, ($C_5$-$C_9$)-Cycloalkenyl, ($C_5$-$C_7$)-Cycloalkyl-($C_1$-$C_4$)-alkyl. Aryl oder teilhydriertes Aryl, das jeweils durch ($C_1$-$C_2$)-Alkyl, ($C_1$-$C_2$)-Alkoxy oder Halogen substituiert sein kann, Aryl ($C_1$-$C_4$)alkyl, dessen Arylrest, wie vorstehend definiert, substituiert sein kann, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden Aminosäure,

$R^5$ = Wasserstoff, ($C_1$-$C_6$)-Alkyl, ($C_2$-$C_6$)-Alkenyl oder Aryl-($C_1$-$C_4$)-alkyl,

Y' = Wasserstoff oder Hydroxy,

Z' = Wasserstoff oder

Y' und Z' = zusammen Sauerstoff,

X' = ($C_1$-$C_6$)-Alkyl, ($C_2$-$C_6$)-Alkenyl, ($C_5$-$C_9$)-Cycloalkyl, Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)alkyl-amino oder Methylendioxy mono-, di- oder trisubstituiert sein kann, oder Indol-3-yl bedeutet, sowie deren physiologisch unbedenkliche Salze.

Verbindungen der Formel VIII können durch N-Acylierung von Verbindungen der Formel IV, in der $R^1$ und $R^2$ die oben genannte Bedeutung haben und R die obige Bedeutung mit Ausnahme der von Wasserstoff hat, mit Verbindungen der Formel Acyl-OH, in der Acyl wie vorstehend definiert ist, und anschließende hydrogenolytische, saure oder basische Abspaltung der Reste R und ggf. $R^5$ hergestellt werden.

Die Kondensation der Verbindungen der Formel Acyl-OH mit den Estern der Formel IV erfolgt vorzugsweise nach bekannten Verfahren der Peptidchemie. Besonders bevorzugt sind solche Verfahren, die ausreichend Schutz vor Racemisierung bieten, wie z. B. die DCC/HOBt-Methode oder die in der DE-OS 2 901 843 beschriebenen Alkanphosphonsäureanhydrid-Methode.

Die Verbindungen der allgemeinen Formel VIII besitzen eine langandauernde, intensive blutdrucksenkende Wirkung. Sie werden nach peroraler Gabe gut resorbiert und können zur Bekämpfung des Bluthochdrucks verschiedener Genese eingesetzt und für sich oder in Kombination mit anderen blutdrucksenkenden, gefäßerweiternden oder diuretisch wirksamen Verbindungen angewandt werden. Die Anwendung kann intravenös, subcutan oder peroral erfolgen, die perorale Gabe wird bevorzugt. Die Dosierung bei peroraler Gabe liegt bei 0,01-10 mg/kg Tag, vorzugsweise 0,05-1,5 mg/kg Tag insbesondere 0,05-0,5 mg/kg Tag.

Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden. Bei intravenöser oder subcutaner Verabreichung sollte die Einzeldosis zwischen 0,1 und 250 µg/kg Tag liegen.

Die folgenden Beispiele sollen die Erfindung erläutern.

## Beispiel 1

2β,3aα,7aβ-Octahydroindol-2-carbonsäure

a) trans-1-Acetamido-2-chlormercurio-cyclohexan

Zu einer Suspension von 65 g (0,2 Mol) Quecksilber (II)-nitrat in 150 ml Acetonitril werden bei 0 °C im Verlauf von 20 Minuten 20 ml (0,2 Mol) Cyclohexen in 50 ml Acetonitril getropft. Nach 1 Stunde bei Raumtemperatur wird die gelbe Lösung auf eine Mischung aus 100 ml gesättigter Kochsalzlösung und 500 ml Wasser gegossen, das ausgefallene Produkt abgesaugt, mit Wasser gewaschen, und in Vakuum getrocknet. Man erhält 75 g farblose Kristalle vom Schmelzpunkt 200-201 °C.

b) trans-1-Acetamido-2-(2-chlor-2-cyanoethyl)-cyclohexan

31 g trans-1-Acetamido-2-chlormercurio-cyclohexan werden in 250 ml 95 %igem Ethanol suspendiert : 187 ml (4 Equivalente) 2-Chloracrylnitril werden zugesetzt. Unter Eiskühlung gibt man eine Lösung von 3 g Natriumborhydrid in 50 ml Ethanol so schnell wie möglich zu. Nach Aufwärmen auf Raumtemperatur wird das ausgeschiedene elementare Quecksilber über Kieselgur abgesaugt, mit Ethanol gut nachgewaschen und das Filtrat eingeengt. Der Rückstand wird in Methylenchlorid aufgenommen. zur Zerstörung der Borsäureester dreimal mit 1 N Natronlauge gewaschen, über Natriumsulfat getrocknet und eingeengt. Man erhält 13 g der Titelverbindung als gelbes Öl.

c) 1-Acetyl-2-cyano-trans-octahydroindol

3,2 g trans-1-Acetamido-2-(2-chlor-2-cyanoethyl)-cyclohexan werden in 25 ml Dimethylformamid gelöst und bei 0 °C zu einer Suspension von 0,7 g Natriumhydrid (50 % in Öl, dreimal mit Hexan gewaschen) in 10 ml Dimethylformamid getropft. Nach 1 Stunde bei Raumtemperatur wird auf Wasser gegossen, mit 5 N Salzsäure sauer gestellt und mit Methylenchlorid extrahiert. Der Extrakt wird viermal mit Wasser gewaschen, über Natriumsulfat getrocknet und eingeengt. Das Rohprodukt enthält ein Gemisch aus 18,5 Teilen des 2β,3aα,7aβ-Isomeren und 1 Teil des 2β,3aβ,7aα-Isomeren. Beim Verreiben mit Diisopropylether kristallisieren 0,7 g des 2β,3aα,7aβ-Isomeren aus. Chromatographie der Mutterlauge an Kieselgel mit Essigester/Cyclohexan (2 : 1) als Laufmittel liefert noch einmal 0,2 g des 2β,3aα,7aβ-Isomeren und 45 mg des 2β,3aβ,7aα-Isomeren.
Physikalische Daten :

2β,3aα,7aβ-Isomeres :

Schmp. 110-113 °C
$^1$H-NMR-Daten (270 MHz, DMSO-$d_6$, 100 °C) : δ = 4,90 (d.J = 5Hz, 1H) ; 3,05 (dt, $J_1$ = 5 Hz, $J_2$ = 1,5 Hz, 1H) ; 2,60 (d, 1H) ; 2,17 (d, 1H) ; 2,03 (s, 3H) ; 1,95 (br, d, 1H) ; 1,85-1,6 (m, 4H) ; 1,4-1,0 (m, 4H) ppm.MS (m/e) : 192 ($M^+$, 32 %) ; 150 (M—$CH_2$=C=O, 59 %) ; 149 (18 %) ; 139 (14 %) 107 (100 %) ; 95 (15 %) ; 43 (39 %).

2β, 3aβ,7aα-Isomeres :

Öl
$^1$H-NMR-Daten (270 MHz, DMSO-$d_6$, 100 °C) : δ = 4,66 (t, J = 5Hz, 1H) ; 3,10-3,0 (m, 1H) ; 2,75-2,6 (m, 1H) ; 2,08 (s, 3H) ; 2,0-1,4 (m, 5H) : 1,4-1,2 (m, 4H) ppm.

d) 2β,3aα,7aβ-Octahydroindol-2-carbonsäure

2,8 g 2β,3aα,7aβ-1-Acetyl-2-cyano-octahydroindol werden mit 30 ml 5 N Salzsäure 4 Stunden am Rückfluß gekocht, zur Trockne eingeengt, mit Wasser aufgenommen, mit schwach basischem Ionenaustauscher (Amberlite® IRA 93, OH-Form) auf pH4,7 gestellt, abfiltriert, eingeengt, mit Ethanol aufgenommen und mit Aceton gefällt. Absaugen und Trocknen liefert 1,9 g Titelverbindung vom Schmelzpunkt 286-288 °C (Zers.).
$^1$H-NMR-Daten ($D_2O$, 270 MHz) : δ = 4,15 (d, J = 5Hz, 1H) ; 2,88 (dd, $J_1$ = 12 Hz, $J_2$ = 3,8 Hz, 1H) ; 2,55 (mc, 1H) ; 2,3-1,1 (m, 10H) ppm.

## Beispiel 2

2β,3aα,7aβ-Octahydrocyclopenta[b]pyrrol-2-carbonsäure

a) trans-1-Acetamido-2-chlormercurio-cyclopentan

Eine Suspension von 38,3 g Quecksilber (II) nitrat in 80 ml Acetonitril wird bei 0 °C tropfenweise mit 10 ml Cyclopenten in 30 ml Acetonitril versetzt. Nach 1 Stunde bei Raumtemperatur wird auf eine Mischung von 60 ml gesättigter Kochsalzlösung und 250 ml Wasser gegossen, abgesaugt, mit Wasser gewaschen und getrocknet. Man erhält 26 g der Titelverbindung als amorphes Pulver.

b) trans-1-Acetamido-2-(2-chlor-2-cyanoethyl)-cyclopentan

16,1 g trans-1-Acetamido-2-chlormercurio-cyclopentan werden in 200 ml Ethanol suspendiert. 10,7 ml 2-Chloracrylnitril werden zugesetzt, danach so schnell wie möglich unter Eiskühlung 1,7 g Natriumborhydrid in 40 ml Ethanol. Nach 1 Stunde bei Raumtemperatur wird über Kieselgur abgesaugt, eingeengt, mit Methylenchlorid aufgenommen, mit 1 N Natronlauge zweimal gewaschen, über Natriumsulfat getrocknet, eingeengt. Chromatographie an Kieselgel (Laufmittel Essigester) ergibt 2,2 g kristallines Produkt, Schmp. 114-117 °C.

$^1$H-NMR-Daten ($CDCl_3$) : δ = 5,4 (br. s, 1H) ; 4,67 (t, J = 7Hz, 1H) ; 3,95 (mc, 1H) ; 2,6-1,0 (m, 9H) ; 1,97 (s, 3H) ppm MS (m/e) : 214 ($M^+$, 8 %) ; 179 (10 %) ; 140 (20 %) ; 137 (35 %) ; 98 (48 %) ; 60 (78 %) ; 56 (100 %), 43 (68 %).

c) 1-Acetyl-2-cyano-trans-octahydrocyclopenta[b]pyrrol

0,75 g trans-1-Acetamido-2-(2-chlor-2-cyanoethyl)cyclopentan werden in 20 ml DMF-gelöst zu 200 mg Natriumhydrid (50 % in Öl, zweimal mit Hexan gewaschen) gegeben und 1 Stunde bei Raumtemperatur gerührt. Nach Eingießen in 1 N Salzsäure wird mit Methylenchlorid extrahiert, die organische Phase viermal mit Wasser gewaschen, getrocknet und eingeengt. Durch Chromatographie an Kieselgel mit Essigester/Cyclohexan (2 : 1) als Laufmittel erhält man 0,12 g 2β,3aβ,6aα-Isomeres als Öl und 0,45 g 2β,3aα,6aβ-Isomeres als farblose Kristalle vom Schmelzpunkt 115-117 °C.

$^1$H-NMR-Daten ($CDCl_3$) : δ = 5,02 (d, J = 7,5 Hz, 1H) ; 3,33 (dt, $J_1$ = 11Hz, $J_2$ = 6 Hz, 1H) ; 2,9-1,0 (m, 9H) ; 2,07 (s, 3H) ppm.

d) 2β,3aα,6aβ-Octahydrocyclopenta[b]pyrrol-2-carbonsäure

0,45 g 2β,3aα,6aβ-1-Acetyl-2-cyano-octahydrocyclopenta[b]-pyrrol werden mit 5 ml 5 N Salzsäure 3 Stunden am Rückfluß erhitzt, zur Trockne eingeengt, mit Wasser aufgenommen, mit schwach basischen Ionenaustauscher (Amberlite® IRA 93, OH-Form) auf pH 6 gestellt, abfiltriert, eingeengt und mit Ethanol/Ether kristallisiert.

Ausbeute : 0,26 g

Schmp. : > 250 °C (Zers.)

$^1$H-NMR-Daten ($D_2O$) : δ = 4,5 (t, 1H) ; 3,6-3,0 (m, 1H) ; 2,6-1,0 (m, 9H) ppm.MS (m/e) ; 155 ($M^+$, 1 %) ; 154 ($M^+$—H, 3 %) : 110 ($M^+$—COOH, 100 %) ; 67 (24 %).

## Beispiel 3

1-Acetyl-2-cyano-4,5-cis-diethyl-pyrrolidin

a) erythro-3-Acetamido-4-chlormercurio-hexan

39 g Quecksilber (II)-nitrat werden in 50 ml Acetonitril suspendiert, bei 0 °C werden 10 g trans-3-Hexen in 20 ml Acetonitril zugetropft. Nach 45 Minuten bei Raumtemperatur wird auf 500 ml gesättigte Natriumchlorid-Lösung gegossen. Die Titelverbindung scheidet sich zunächst ölig ab, sie kristallisiert nach kurzer Zeit durch. Schmp. Ausbeute 41,5 g.

b) 2-Chlor-threo-5-acetamido-4-ethyl-heptan-nitril

15 g erythro-3-Acetamido-4-chlormercurio-hexan und 9.6 ml 2-Chloracrylnitril werden in 250 ml Ethanol gelöst. Unter Eiskühlung gibt man 1.52 g Natriumborhydrid schnell zu. Nach 45 Minuten wird das gebildete elementare Quecksilber durch Kieselgur abgesaugt, das Filtrat eingeengt, mit Methylenchlorid aufgenommen, dreimal mit 1 N Natronlauge gewaschen, über Magnesiumsulfat getrocknet und eingeengt. Man erhält 6,6 g eines 1 : 1-Isomerengemisches in der 2-Position.

c) 1-Acetyl-2-cyano-4,5-cis-diethyl-pyrrolidin

6,6 g 2-Chlor-threo-5-acetamido-4-ethyl-heptan-nitril werden in 100 ml Dimethylformamid gelöst und bei 0 °C zu 1,7 g Natriumhydrid (50 % in Öl, 2 × mit Hexan gewaschen) getropft. Nach 2 Stunden bei Raumtemperatur wird auf 1 N Salzsäure gegossen und mit Methylenchlorid zweimal extrahiert. Die organische Phase wird viermal mit Wasser gewaschen, getrocknet und eingeengt. Man erhält 4,6 g eines braumen Öls, aus dem durch Säulenchromatographie an Kieselgel mit Essigester/Cyclohexan (2 : 1) als Laufmittel die beiden Isomeren der Titelverbindung isoliert werden.

Isomer 1 (1,63 g)

2β,4β,5β-Form : $R_f$-Wert (Essigester/Cyclohexan 4 : 1) 0,5 : Öl ; $^1$H-NMR-Daten : 5,0-4,4 (m, 1H) ; 4,0-3,3 (m, 1H) ; 2,6-0,7 (m, 13H) ; 2,15 + 2,12 (2s, 3H) ppm.

MS (m/e) : 194 ($M^+$, 8 %), 165 ($M^+$—$C_2H_5$, 4 %) : 123 ($M^+$—$CH_3CO$—$C_2H_5$, 100 %) ; 43 (17 %).

Isomer 2 (1,63 g)

2β,4α,5α-Form : $R_f$-Wert (Essigester/Cyclohexan 4 : 1) 0,43, F. 144-149 °C : $^1$H-NMR-Daten : 5,8-5,4 (m, 1H) ; 4,3-3,9 (m, 1H) ; 3,0-2,5 (m, 1H) ; 2,03 (s, 3H) ; 2,2-1,1 (m, 6H) ; 1,08 + 0,97 (2t, 6H) ppm : MS (m/e) ; 194 ($M^+$, 8 %) ; 165 ($M^+$—$C_2H_5$, 4 %), 123 ($M^+$—$CH_3CO$—$C_2H_5$, 100 %) ; 43 (14 %).

## Beispiel 4

2β,4β, 5β-Diethylprolin

1,63 g 2β,4β,5β-1-Acetyl-2-cyano-4,5-diethyl-pyrrolidin (Beispiel 3c, Isomer 1) werden mit 20 ml 5 N Salzsäure 2,5 Stunden am Rückfluß erhitzt. Nach Einengen zur Trockne wird mit Amberlite® IRA 93 ($OH^-$-Form) auf einen pH-Wert von 6 eingestellt, filtriert, eingeengt, mit Ethanol/Aceton aufgenommen und abgesaugt. Reinigung erfolgt durch Chromatographie an Kieselgel mit Methylenchlorid/Methanol/Eisessig/Wasser (10 : 3 : 1 : 1) als Laufmittel. Man erhält 1,05 g Titelverbindung vom Schmelzpunkt 230-235° (Zers.).

$^1$H-NMR-Daten ($D_2O$) : $\delta = 4{,}1$ (t, J = 9Hz, 1H) ; 3,25 (dt, $J_1 = 7$Hz, $J_2 = 6$ Hz, 1H) : 2,9-1,0 (m, 7H) ; 1,0 (t, J = 7Hz, 6H)ppm.

Ms (m/e) : 171 ($M^+$, 2 %) ; 142 ($M^+$—$C_2H_5$, 100 %) ; 126 ($M^+$—COOH, 74 %) ; 115 (12 %) ; 96 (30 %) ; 83 (10 %) ; 69 (33 %) ; 55 (19 %).

## Beispiel 5

2β-4α,5α-Diethylprolin

1,63 g 2β,4α,5α-1-Acetyl-2-cyano-4,5-diethylpyrrolidin (Beispiel 3c, Isomer 2) werden mit 20 ml 5 N Salzsäure entsprechend den in Beispiel beschriebenen Verfahren umgesetzt. Man erhält 0,72 g der Titelverbindung vom Schmelzpunkt 158-162 °C.

$^1$H-NMR-Daten ($D_2O$) : $\delta = 4{,}15$ (t. J = 8 Hz, 1H) ; 3,68 (q, J = 6Hz, 1H) ; 2,5-1,1 (m, 7H) ; 1,0 (t, J = 7Hz, 6H) ppm.

MS (m/e) : 171 ($M^+$, 1 %) ; 142 ($M^+$—$C_2H_5$, 100 %) : 126 ($M^+$—$CO_2H$, 96 %) : 115 (19 %), 96 (33 %) : 83 (11 %) : 69 (36 %) : 55 (22 %).

## Beispiel 6

1-Acetyl-2-cyano-4,5-cis-dimethyl-pyrrolidin

Hergestellt aus trans-2-Buten analog den in Beispiel 3 a bis c beschriebenen Verfahrensweisen.

## Beispiel 7

4,5-cis-Dimethylprolin

Hergestellt aus 1-Acetyl-2-cyano-4,5-cis-dimethyl-pyrrolidin analog der in Beispiel 4 beschriebenen Verfahrensweise.

## Beispiel 8

1-Acetyl-2-cyano-4,5-trans-dimethyl-pyrrolidin

Hergestellt aus cis-2-Buten analog den in Beispiel 3a bis c beschriebenen Verfahrensweisen.

## Beispiel 9

4,5-trans-Dimethylprolin

Hergestellt aus 1-Acetyl-2-cyano-4,5-trans-dimethyl-pyrrolidin analog der in Beispiel 4 beschriebenen Verfahrensweisen.

## Beispiel 10

2β,3aα,8aβ-Decahydrocyclohepta[b]pyrrol-2-carbonsäure

Hergestellt aus Cyclohepten analog den in Beispiel 1a bis d beschriebenen Verfahrensweisen.

Beispiel 11

2β,3aα,9aβ-Decahydrocycloocta[b]pyrrol-2-carbonsäure

Hergestellt aus Cycloocten analog den in Beispiel 1a bis d beschriebenen Verfahrensweisen.

**Patentansprüche** (für die Vertragsstaaten : BE, CH, DE, FR, GB, IT, LI, LU, NL, SE)

1. Verbindung der Formel I

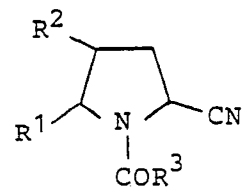

(I)

in welcher

$R^1$ und $R^2$ gleich oder verschieden sind und für geradkettiges oder verzweigtes Alkyl mit 1 bis 6 C-Atomen oder für ($C_6$ bis $C_{10}$)-Aryl, stehen oder gemeinsam für eine der Ketten $-(CH_2)_n-$ oder $(-CH_2)_p-CH=CH-(CH_2)_q-$ stehen, wobei n, p und q jeweils ganzzahlig, n = 3 — 6 und (p + q) = 1 — 4 sind, und

$R^3$ geradkettiges oder verzweigtes Alkyl mit 1 bis 5 C-Atomen oder ($C_6$ bis $C_{10}$)-Aryl, vorzugsweise Phenyl bedeutet,

1-Acetyl-2-cyano-2β,3aβ,7aβ-octahydroindol, N-Acetyl-2-cyano-2β,3aβ,6aβ-octahydrocyclopenta[b]pyrrol und N-Acetyl-2-cyano-2β,3aβ,8aβ-decahydrocyclohepta[b]pyrrol ausgenommen.

2. Verbindung der Formel I gemäß Anspruch 1, in welcher $R^1$ und $R^2$ eine der im Anspruch 1 definierten Ketten bilden und zueinander trans-konfiguriert sind.

3. Verfahren zur Herstellung einer Verbindung der Formel I gemäß Anspruch 1 oder 2, dadurch gekennzeichnet, daß man eine Organoquecksilberverbindung der Formel II

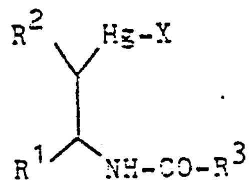

(II)

in welcher $R^1$, $R^2$ und $R^3$ die in Anspruch 1 definierte Bedeutung haben und X Halogen, Pseudohalogen oder Acetat bedeutet, mit 2-Chloracrylnitril und Natriumborhydrid oder Kaliumborhydrid zu einer Verbindung der Formel III

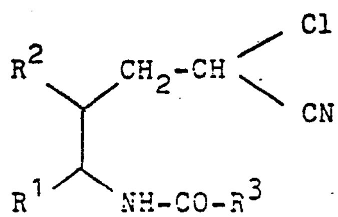

(III)

in welcher $R^1$, $R^2$ und $R^3$ die im Anspruch 1 definierte Bedeutung haben, umsetzt, diese durch Umsetzung mit einer Base in einem aprotischen Lösungsmittel gegebenenfalls unter Zusatz eines Phasentransferkatalysators zur Verbindung der Formel I cyclisiert.

4. Verbindung der Formel III, in welcher $R^1$, $R^2$ und $R^3$ die im Anspruch 1 definierte Bedeutung haben.

5. Verwendung einer Verbindung der Formel I gemäß einem der Ansprüche 1 oder 2 zur Herstellung einer Verbindung der Formel IV

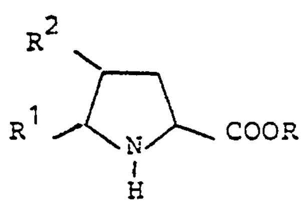

(IV)

in welcher $R^1$ und $R^2$ die im Anspruch 1 genannten Bedeutungen haben und R für Wasserstoff, Alkyl mit 1 bis 10 C-Atomen, Aralkyl mit 7 bis 9 C-Atomen, Cycloalkyl mit 5 bis 10 C-Atomen, Alkylcycloalkyl mit 6 bis 12 C-Atomen oder Cycloalkylalkyl mit 6 bis 12 C-Atomen steht durch Solvolyse mit einer Verbindung der Formel ROH, in welcher R vorstehende Bedeutung hat.

6. Verwendung gemäß Anspruch 5, dadurch gekennzeichnet, daß R Wasserstoff bedeutet.

7. 1-Acetyl-2-cyano-2β,3aα,7aβ-octahydroindol.

8. 1-Acetyl-2-cyano-2β,3aβ,7aα-octahydroindol.

**Patentansprüche** (für den Vertragsstaat AT)

1. Verfahren zur Herstellung einer Verbindung der Formel I

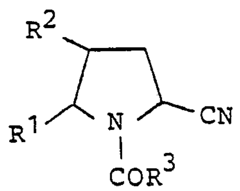

(I)

in welcher

$R^1$ und $R^2$ gleich oder verschieden sind und für geradkettiges oder verzweigtes Alkyl mit 1 bis 6 C-Atomen oder für ($C_6$ bis $C_{10}$)-Aryl, stehen oder gemeinsam für eine der Ketten $-(CH_2)_n-$ oder $-(CH_2)_p-CH=CH-(CH_2)_q-$ stehen, wobei n, p und q jeweils ganzzahlig, $n = 3-6$ und $(p + q) = 1-4$ sind, und

$R^3$ geradkettiges oder verzweigtes Alkyl mit 1 bis 5 C-Atomen oder ($C_6$ bis $C_{10}$)-Aryl, vorzugsweise Phenyl bedeutet,

1-Acetyl-2-cyano-2β, 3aβ, 7aβ-octahydroindol, N-Acetyl-2-cyano-2β, 3aβ, 6aβ-octahydrocyclopenta [b] pyrrol und N-Acetyl-2-cyano-2β, 3aβ, 8aβ-decahydrocyclohepta [b] pyrrol ausgenommen, dadurch gekennzeichnet, daß man eine Organoquecksilberverbindung der Formel II

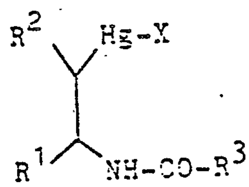

(II)

in welcher $R^1$, $R^2$ und $R^3$ die oben definierte Bedeutung haben und X Halogen, Pseudohalogen oder Acetat bedeutet, mit 2-Chloracrylnitril und Natriumborhydrid oder Kaliumborhydrid zu einer Verbindung der Formel III

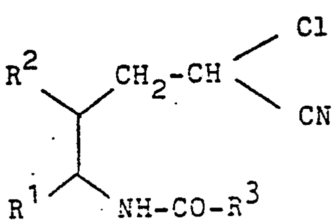

(III)

in welcher $R^1$, $R^2$ und $R^3$ die oben definierte Bedeutung haben, umsetzt, diese durch Umsetzung mit einer Base in einem aprotischen Lösungsmittel gegebenenfalls unter Zusatz eines Phasentransferkatalysators zur Verbindung der Formel I cyclisiert.

2. Verfahren zur Herstellung einer Verbindung der Formel I gemäß Anspruch 1, in welcher $R^1$ und $R^2$ eine der im Anspruch 1 definierten Ketten bilden und zueinander trans-konfiguriert sind.

3. Verfahren zur Herstellung einer Verbindung der Formel III, in welcher $R^1$, $R^2$ und $R^3$ wie im Anspruch 1 definiert sind, dadurch gekennzeichnet, daß man eine Organoquecksilberverbindung der Formel II, in welcher $R^1$, $R^2$ und $R^3$ die in Anspruch 1 definierte Bedeutung haben und X Halogen, Pseudohalogen oder Acetat bedeutet, mit 2-Chloracrylnitril und Natriumborhydrid oder Kaliumborhydrid umsetzt.

4. Verwendung einer Verbindung der Formel I gemäß einem der Ansprüche 1 oder 2 zur Herstellung einer Verbindung der Formel IV

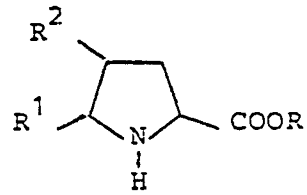

(IV)

in welcher $R^1$ und $R^2$ die im Anspruch 1 genannten Bedeutungen haben und R für Wasserstoff, Alkyl mit 1 bis 10 C-Atomen, Aralkyl mit 7 bis 9 C-Atomen, Cycloalkyl mit 5 bis 10 C-Atomen, Alkylcycloalkyl mit 6 bis 12 C-Atomen oder Cycloalkylalkyl mit 6 bis 12 C-Atomen steht durch Solvolyse mit einer Verbindung der Formel ROH, in welcher R vorstehende Bedeutung hat.

5. Verwendung gemäß Anspruch 4, dadurch gekennzeichnet, daß R Wasserstoff bedeutet.

6. Verfahren gemäß Anspruch 1 zur Herstellung von 1-Acetyl-2-cyano-2β, 3aα, 7aβ-octahydroindol.

7. Verfahren gemäß Anspruch 1 zur Herstellung von 1-Acetyl-2-cyano-2β, 3aβ, 7aα-octahydroindol.

**Claims** (for the Contracting States : BE, CH, DE, FR, GB, IT, LI, LU, NL, SE)

1. A compound of the formula I

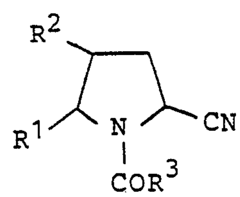

(I)

in which

$R^1$ and $R^2$ are identical or different and represent straight-chain or branched alkyl having 1 to 6 carbon atoms or represent ($C_6$ to $C_{10}$)-aryl, or together represent one of the chains $-(CH_2)_n-$ or $-(CH_2)_p-CH=CH-(CH_2)_q$, n, p and q each being an integer, n being 3 — 6 and (p + q) being 1 — 4, and

$R^3$ denotes straight-chain or branched alkyl having 1 to 5 carbon atoms or ($C_6$ to $C_{10}$)-aryl, preferably phenyl, with the proviso that 1-acetyl-2-cyano-2β, 3aβ, 7aβ-octahydroindole, N-acetyl-2-cyano-2β-3aβ, 6aβ-octahydrocyclopenta [b]-pyrrole and N-acetyl-2-cyano-2β, 3aβ, 8aβ-decahydrocyclohepta-[b] pyrrole are excluded.

2. A compound of the formula I as claimed in claim 1, in which $R^1$ and $R^2$ form one of the chains defined in claim 1 and have the trans configuration with respect to one another.

3. A process for the preparation of a compound of the formula I as claimed in claim 1 or 2, which comprises reacting an organomercury compound of the formula II

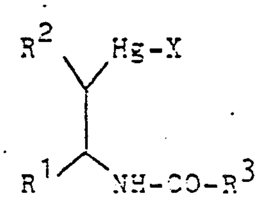

(II)

in which $R^1$, $R^2$ and $R^3$ have the meaning defined in claim 1 and X denotes halogen, pseudohalogen or acetate, with 2-chloroacrylonitrile and sodium borohydride or potassium borohydride to give a compound of the formula III

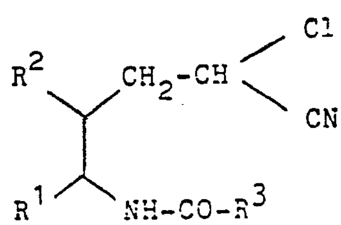

(III)

in which $R^1$, $R^2$ and $R^3$ have the meaning defined in claim 1, and cyclizing the latter to the compound of the formula I by reaction with a base in an aprotic solvent, optionally with the addition of a phase-transfer catalyst.

4. A compound of the formula III, in which $R^1$, $R^2$ and $R^3$ have the meaning defined in claim 1.

5. The use of a compound of the formula I as claimed in one of claims 1 or 2 for the preparation of a compound of the formula IV

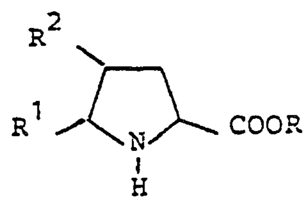

(IV)

in which $R^1$ and $R^2$ have the meanings mentioned in claim 1, and R represents hydrogen, alkyl having 1 to 10 carbon atoms, aralkyl having 7 to 9 carbon atoms, cycloalkyl having 5 to 10 carbon atoms, alkylcycloalkyl having 6 to 12 carbon atoms or cycloalkylalkyl having 6 to 12 carbon atoms, by solvolysis with a compound of the formula ROH in which R has the above meaning.

6. The use as claimed in claim 5, wherein R denotes hydrogen.

7. 1-Acetyl-2-cyano-2β, 3aα, 7aβ-octahydroindole.

8. 1-Acetyl-2-cyano-2β, 3aβ, 7aα-octahydroindole.

**Claims** (for the Contracting State AT)

1. A process for the preparation of a compound of the formula I

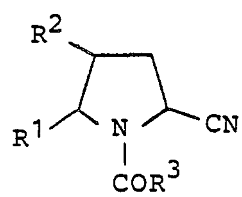

(I)

in which

$R^1$ and $R^2$ are identical or different and represent straight-chain or branched alkyl having 1 to 6 carbon atoms or represent ($C_6$ to $C_{10}$)-aryl, or together represent one of the chains $-(CH_2)_n-$ or $-(CH_2)_p-CH=CH-(CH_2)_q$, n, p and q each being an integer, n being 3 — 6 and (p + q) being 1 — 4, and

$R^3$ denotes straight-chain or branched alkyl having 1 to 5 carbon atoms or ($C_6$ to $C_{10}$)-aryl, preferably phenyl with the proviso that 1-acetyl-2-cyano-2β,3aβ,7aβ-octahydroindoleN-acetyl-2-cyano-2β,3aβ,6aβ-octahydrocyclopenta[b]-pyrrole and N-acetyl-2-cyano-2β, 3aβ, 8aβ-decahydrocyclohepta-[b] pyrrole are excluded, which comprises reacting an organomercury compound of the formula II

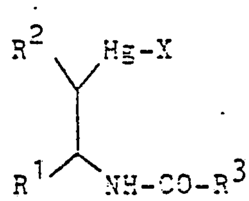

(II)

in which $R^1$, $R^2$ and $R^3$ have the meaning defined in claim 1 and X denotes halogen, pseudohalogen or acetate, with 2-chloroacrylonitrile and sodium borohydride or potassium borohydride to give a compound of the formula III

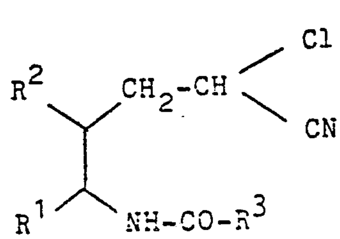

(III)

in which $R^1$, $R^2$ and $R^3$ have the meaning defined in claim 1, and cyclizing the latter to the compound of the formula I by reaction with a base in an aprotic solvent, optionally with the addition of a phase-transfer catalyst.

2. A process for the preparation of a compound of the formula I as claimed in claim 1, in which $R^1$ and $R^2$ form one of the chains defined in claim 1 and have the trans configuration with respect to one another.

3. A process for the preparation of a compound of the formula III, in which $R^1$, $R^2$ and $R^3$ are as defined in claim 1, characterized in that an organomercury compound of the formula II, in which $R^1$, $R^2$ and $R^3$ are as defined in claim 1 and X denotes halogen, pseudohalogen or acetate, is reacted with 2-chloroacrylonitrile and sodium borohydride or potassium borohydride.

4. The use of a compound of the formula I as claimed in one of claims 1 or 2 for the preparation of a compound of the formula IV

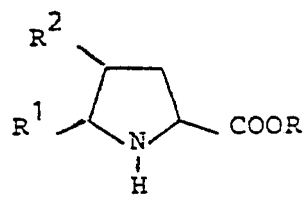

(IV)

in which $R^1$ and $R^2$ have the meanings mentioned in claim 1, and R represents hydrogen, alkyl having 1 to 10 carbon atoms, aralkyl having 7 to 9 carbon atoms, cycloalkyl having 5 to 10 carbon atoms, alkylcycloalkyl having 6 to 12 carbon atoms or cycloalkylalkyl having 6 to 12 carbon atoms, by solvolysis with a compound of the formula ROH in which R has the above meaning.

5. The use as claimed in claim 4, wherein R denotes hydrogen.

6. The process as claimed in claim 1 for the preparation of 1-acetyl-2-cyano-2β, 3aα, 7aβ-octahydroindole.

7. The process as claimed in claim 1 for the preparation of 1-acetyl-2-cyano-2β, 3aβ, 7aα-octahydroindole.

**Revendications** (pour les Etats contractants : BE, CH, DE, FR, GB, IT, LI, LU, NL, SE)

1. Composé répondant à la formule I

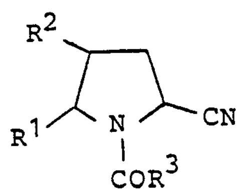

(I)

dans laquelle

$R^1$ et $R^2$ sont identiques ou différents et représentent un groupe alcoyle linéaire ou ramifié en $C_1$ à $C_6$ ou un groupe aryle en $C_6$ à $C_{10}$, ou forment ensemble une des chaînes $-(CH_2)_n-$ ou $-(CH_2)_p-CH=CH-(CH_2)_q$, n, p et q étant respectivement des nombres entiers, n = 3 — 6 et (p + q) = 1 — 4, et

$R^3$ représente un groupe alcoyle linéaire ou ramifié en $C_1$ à $C_5$ ou un groupe aryle en $C_6$ à $C_{10}$, de préférence un groupe phényle,

à l'exception des composés 1-acétyl-2-cyano-2β, 3aβ, 7aβ-octahydroindole, N-acétyl-2-cyano-2β, 3aβ, 6aβ-octahydrocyclopenta-[b]-pyrrole et N-acétyl-2-cyano-2β, 3aβ, 8aβ-décahydrocyclohepta-[b]-pyrrole.

2. Composé répondant à la formule I suivant la revendication 1, dans laquelle $R^1$ et $R^2$ forment une des chaînes définies dans la revendication 1 et sont dans la configuration trans l'un par rapport à l'autre.

3. Procédé de préparation d'un composé répondant à la formule I suivant la revendication 1 ou 2, caractérisé en ce qu'on fait réagir un composé organomercurique répondant à la formule II

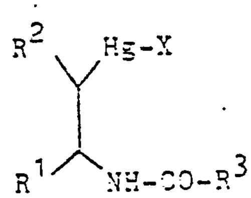

(II)

dans laquelle $R^1$, $R^2$ et $R^3$ ont la signification définie ci-dessus et X représente un atome d'halogène, un pseudohalogène ou un groupe acétate, avec du 2-chloroacrylonitrile et du borohydrure de sodium ou de borohydrure de potassium pour donner un composé répondant à la formule III

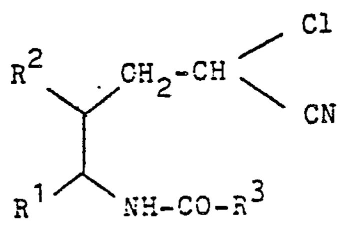

(III)

dans laquelle $R^1$, $R^2$ et $R^3$ ont la signification définie dans la revendication 1, on cyclise ce composé par réaction avec une base dans un solvant aprotique, en ajoutant le cas échéant un catalyseur à transfert de phase, pour donner un composé répondant à la formule I.

4. Composé répondant à la formule III, dans laquelle $R^1$, $R^2$ et $R^3$ ont la signification définie dans la revendication 1.

5. Utilisation d'un composé répondant à la formule I définie à la revendication 1 ou 2 pour la préparation d'un composé répondant à la formule IV

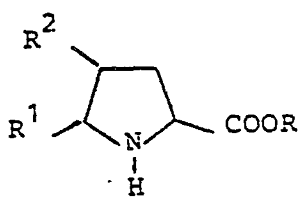

(IV)

dans laquelle $R^1$ et $R^2$ ont les significations indiquées dans la revendication 1 et R représente un atome d'hydrogène, un groupe alcoyle en $C_1$ à $C_{10}$, un groupe aralcoyle en $C_7$ à $C_9$, un groupe cycloalcoyle en $C_5$ à $C_{10}$, un groupe alcoylcycloalcoyle en $C_6$ à $C_{12}$ ou un groupe cycloalcoylalcoyle en $C_6$ à $C_{12}$, par solvolyse avec un composé répondant à la formule ROH, dans lequel R a la signification précédente.

6. Utilisation suivant la revendication 5, caractérisée en ce que R désigne un atome d'hydrogène.

7. Le composé 1-acétyl-2-cyano-2β,3aα, 7aβ-octahydroindole.

8. Le composé 1-acétyl-2-cyano-2β,3aβ, 7aα-octahydroindole.

**Revendications** (pour l'Etat contractant AT)

1. Procédé de préparation d'un composé répondant à la formule I

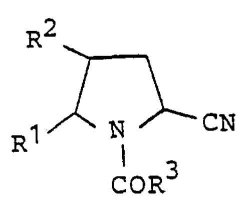

(I)

dans laquelle

$R^1$ et $R^2$ sont identiques ou différents et représentent un groupe alcoyle linéaire ou ramifié en $C_1$ à $C_6$ ou eun groupe aryle en $C_6$ à $C_{10}$, ou forment ensemble une des chaînes $-(CH_2)_n-$ ou $-(CH_2)_p-CH=CH-(CH_2)_q$, n, p et q étant respectivement des nombres entiers, n = 3 — 6 et (p + q) = 1 — 4, et

$R^3$ représente un groupe alcoyle linéaire ou ramifié en $C_1$ à $C_5$ ou un groupe aryle en $C_6$ à $C_{10}$, de préférence un groupe phényle,

à l'exception des composés 1-acétyl-2-cyano-2β, 3aβ, 7aβ-octahydroindole, N-acétyl-2-cyano-2β, 3aβ, 6aβ-octahydrocyclopenta-[b]-pyrrole et N-acétyl-2-cyano-2β,3aβ,8aβ-décahydrocyclohepta-[b]-pyrrole, caractérisé en ce qu'on fait réagir un composé organomercurique répondant à la formule II

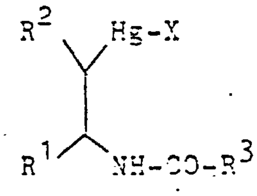

(II)

dans laquelle $R^1$, $R^2$ et $R^3$ ont la signification définie ci-dessus et X représente un atome d'halogène, un pseudohalogène ou un groupe acétate, avec du 2-chloroacrylonitrile et du borohydrure de sodium ou du borohydrure de potassium pour donner un composé répondant à la formule III

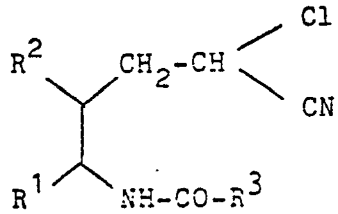

(III)

dans laquelle $R^1$, $R^2$ et $R^3$ ont la signification définie ci-dessus, on cyclise ce composé par réaction avec une base dans un solvant aprotique, en ajoutant le cas échéant un catalyseur à transfert de phase, pour donner un composé répondant à la formule I.

2. Procédé de préparation d'un composé répondant à la formule I suivant la revendication 1, dans laquelle $R^1$ et $R^2$ forment une des chaînes définies dans la revendication 1 et sont dans la configuration trans l'un par rapport à l'autre.

3. Procédé de préparation d'un composé répondant à la formule III, dans laquelle $R^1$, $R^2$ et $R^3$ ont la signification définie dans la revendication 1, caractérisé en ce qu'on fait réagir un composé organomercurique répondant à la formule II dans laquelle $R^1$, $R^2$ et $R^3$ ont la signification définie dans la revendication 1 et X représente un atome d'halogène, un pseudohalogène ou un groupe acétate, avec du 2-chloroacrylonitrile et du borohydrure de sodium ou du borohydrure de potassium.

4. Utilisation d'un composé répondant à la formule I définie à la revendication 1 ou 2 pour la préparation d'un composé répondant à la formule IV

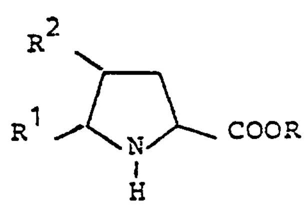

(IV)

dans laquelle $R^1$ et $R^2$ ont les significations indiquées dans la revendication 1 et R représente un atome d'hydrogène, un groupe alcoyle en $C_1$ à $C_{10}$, un groupe aralcoyle en $C_7$ à $C_9$, un groupe cycloalcoyle en $C_5$ à $C_{10}$, un groupe alcoylcycloalcoyle en $C_6$ à $C_{12}$ ou un groupe cycloalcoylalcoyle en $C_6$ à $C_{12}$, par solvolyse avec un composé répondant à la formule ROH, dans lequel R a la signification précédente.

5. Utilisation suivant la revendication 4, caractérisée en ce que R désigne un atome d'hydrogène.

6. Procédé suivant la revendication 1 pour la préparation du 1-acétyl-2-cyano-2β, 3aα, 7aβ-octahydroindole.

7. Procédé suivant la revendication 1 pour la préparation du 1-acétyl-2-cyano-2β, 3aβ, 7aα-octahydroindole.